(19) Europäisches Patentamt
European Patent Office
Office européen des brevets

(11) Publication number: **0 392 021 B1**

(12) **EUROPEAN PATENT SPECIFICATION**

(45) Date of publication of patent specification: **14.09.94**

(51) Int. Cl.[5]: **C12Q 1/26**, C12Q 1/48, C12Q 1/40, C12Q 1/34, C12Q 1/46

(21) Application number: **89907837.2**

(22) Date of filing: **29.06.89**

(86) International application number: **PCT/JP89/00652**

(87) International publication number: **WO 90/00202 (11.01.90 90/02)**

# (54) METHOD FOR ANALYZING COMPONENTS.

(30) Priority: **29.06.88 JP 161148/88**

(43) Date of publication of application: **17.10.90 Bulletin 90/42**

(45) Publication of the grant of the patent: **14.09.94 Bulletin 94/37**

(84) Designated Contracting States: **DE FR GB IT**

(56) References cited:
**GB-A- 2 026 692**
**US-A- 3 627 645**

**H.U. BERGMEYER, "Methods of enzymatic analysis", 3rd edition, vol. VIII, VCH Verlagsgesmbh, Weinheim (DE), 1985; pp. 521-529&NUM;**

**H.U. BERGMEYER, "Methods of enzymatic analysis", 3rd edition, vol. VII, VCH Verlagsgesmbh, Weinheim (DE), 1985; pp. 251-279&NUM;**

**JOURNAL OF BIOLOGICAL CHEMISTRY, vol. 243, 1968; pp. 4083-4088&NUM;**

**FEBS LETTERS, vol. 34, 1973; pp. 143-144&NUM;**

(73) Proprietor: **KYOWA MEDEX CO. LTD.**
**No. 6-1, Ote-machi 1-chome Chiyoda-ku**
**Tokyo-to (JP)**

(72) Inventor: **MIIKE, Akira,410-1, Nameri**
**Nagaizumi-cho,Sunto-gun**
**Shizuoka-ken 411 (JP)**
Inventor: **TATANO, Toshio**
**2297-6, Ooka,Numazu-shi**
**Shizuoka-ken 410 (JP)**

(74) Representative: **Casalonga, Axel et al**
**BUREAU D.A. CASALONGA - JOSSE**
**Morassistrasse 8**
**D-80469 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

Industrially Applicable Field

The present invention relates to a method for quantitative determination of a component in a sample by utilizing a reaction system which forms NAD(P)H, wherein NAD(P)H present in the sample or derived from components other than the specific component by reactions is eliminated prior to the quantitative determination of the specific component by utilizing the system which forms NAD(P)H.

Background Art

Several methods for determining a component in a sample by utilizing oxidor eductase which catalyzes a reaction involving NAD(P)-NAD(P)H are known.

The methods are advantageous in stoichiometrical accuracy, small influence of other components, and the like. However, when NAD(P)H derived from components other than the specific component (hereinafter referred to as other components) is added during the determination, such NAD(P)H must be avoided or removed.

To avoid or remove such NAD(P)H, there is a method in which a reagent blank test is separately carried out and after determination of other components (so called blank test of a sample), the sum of NAD(P)H derived from the other components and the specific component is determined and the amount of the specific component is determined by calculation.

For example, in the case of determination of amylase activity in blood, the enzyme reaction described below is carried out and the rate of formation of NAD(P)H is determined, whereby the amylase activity can be determined. However, as maltose and glucose are contained in blood, NAD(P)H must be determined after they are removed or decomposed.

$$\text{maltopentose} \xrightarrow{\text{amylase}} \text{maltose} + \text{maltotriose} \quad \cdots (1)$$

$$\text{maltose} + \text{Pi} \xrightarrow{\text{maltose phosphorylase}} \text{glucose-1-P} + \text{glucose} \quad \cdots (2)$$

$$\text{glucose} + \text{ATP} \xrightarrow{\text{glucokinase}\cdot\text{Mg}^{++}} \text{glucose-6-P} + \text{ADP} \quad \cdots (3)$$

$$\text{glucose-6-P} + \text{NADP} \xrightarrow{\text{G6PDH}} \text{gluconolactone-6-P} + \text{NADPH} \quad \cdots (4)$$

In the quantitative determination of NAD(P)H by colorimetry of the reaction solution, the absorbancy of the specific component is added to those of the other components, and the determinable range becomes narrow, depending upon the amount of glucose or maltose in a sample. The reason is that the absorbancy which is detectable with a spectrophotometer according to the currently employed technique is limited to a certain range; that is, it is impossible to detect an absorbancy of 3.0 ABS or more with an ordinary spectrophotometer. In view of this aspect, a region in which a measurement value is low is preferred. Further, in the case of reagent blank test with a high absorbancy, reproducibility is poor. Assuming that blank solutions have absorbancies of, for example, A and B (A << B) respectively, the absorbancy E(E << B) appearing as the result of reaction is added to form A + E and D + E (A + E << B + E). When this run is repeated with measurements, the respective variations referred to as As and Bs are obtained (variations in the blank are similarly referred to as Ab and Bb, respectively). From the nature of a spectrophotometer as an apparatus, the percent transmission (T%) is logarithmically converted to absorbancy. Thus, a difference based on the logarithmic function appears on the variation in absorbancy in the

case of low absorbancy (high percent transmission) and on the variation in absorbancy in the case of high absorbancy (low percent transmission), and even though the variations in percent transmission are identical, As < Bs (Ab < Bb) (absorbancy = log $I_0/I$, wherein I is transmittance). From the foregoing, the difference E is calculated for each of the blanks A and B.

With respect to A:

$$(A + E) \pm As - A \pm Ab = E \pm As \pm Ab$$

With respect to B:

$$(B + E) \pm Bs - B \pm Bb = E \pm Bs \pm Bb$$

From As < Bs and Ab < Bb is led As + Ab < Bs + Bb; that is, when the blank has a large value, the variation becomes large, so that the coefficient of variation obtained by division by E becomes large.

An object of the present invention is to provide a method for quantitative determination of the specific component utilizing an NAD(P)H-forming reaction system without performing a blank test of the sample, by eliminating NAD(P)H formed from the other components or present in the sample by reactions.

Disclosure of the Invention

According to the present invention, the specific component in a sample can be quantitatively determined by converting NAD(P)H present in a sample or formed from components other than specific component by reactions into NAD(P) by the action of glutathione of oxidation type and glutathione reductase (first reaction); decomposing the remaining glutathione of oxydation type by the action of γ-glutamyl transpeptidase in the presence of glycine, glycylglycine or an equivalent thereof (second reaction); forming NAD(P)H from the specific component in the sample utilizing an NAD(P)H-forming reaction system; and quantitatively determining the formed NAD(P)H.

The specific component includes both a substrate and an activity of an enzyme which participate in a reaction leading to the NAD(P)H-forming reaction system.

GB-A-2 026 692 discloses a method for determining the activity of an enzyme. In the said method NAD(P)H present in the sample or formed from the components other than the specific component is converted into NADH by employing in the final step lactate dehydrogenase. Subsequently lactate dehydrogenase is inhibited.

In this GB-patent application pretreatment of the sample with lactate dehydrogenase (LDH) and pyruvate is carried out to eliminate interfering NADH formed by components other than that to be assayed, and LDH is inhibited by the addition of oxamic acid (inhibitor).

The method of the GB-patent application is disadvantageous in various aspects. For instance, LDH can not eliminate interfering NADPH and oxamic acid may inhibit not only LDH but also desired enzymes.

US-A-3 627 645, which also concerns a method for determining NADPH, discloses that NADPH can be converted into NADP by an enzyme system employing glutathione reductase, glutathione and a dithiobisheterocyclic compound, which is irreversibly converted to a compound having a characteristic ultraviolet spectrum, capable of converting glutathione in the reduced form to glutathione in the oxidized form.

This US-patent discloses the determination of NAD(P) by the glutathione reductase reaction in which G-(OX), i.e. glutathione of oxydation type is used as the substrate. However, this patent does not teach or suggest the use of the glutathione reductase reaction for the pretreatment of a sample to eliminate the interfering NAD(P)H from the sample.

"Method of Enzymatic Analysis" vol. VIII p. 521-529 (1985) discloses the γ-glutamyl transpeptidase reaction in which G(OH), i.e. glutathione of reduction type is used as the substrate. However, it does not teach or suggest the use of the γ-glutamyl transpeptidase reaction for the pretreatment of a sample to decompose G(OH) in the sample.

"Method of Enyymatic Analysis" vol. VII p. 251-279 (1985) discloses that glutathione reductase has a low specific activity compared to other enzymes used for converting NAD(P)H. This document relates to quantitative analysis of NAD(P)H, in which the specific activity of the enzyme for NAD(P)H is very important.

On the other hand, the purpose of the glutathione reductase reaction in the present invention is the decomposition of NAD(P)H. Therefore, characteristics of the enzyme such as broad substrate specifity are more important than the specific activity. Glutathione reductase catalyses the rection with both NADPH and NADH as substrates, and so the enzyme is really suitable for the present invention.

According to page 267, lines 5-13, in particular line 11, glutathione reductase would be specific for NADPH.

However, no evidence for the alleged specificity for NADPH is given.

This alleged specificity is also in contradiction with the disclosure in Journal of Biological Chemistry, **243**, 4083-4088, (1968).

Brief Description of the Drawings

Fig. 1 shows the change in absorption of the reaction solution at 340 nm determined in accordance with the method of the present invention. Fig. 2 shows the change in absorption in the case where NAD(P)H derived from the other components is added.

The present invention is described in detail below.

In the first reaction, G(OX) and glutathione reductase are added to a sample, if necessary, together with a substrate or an enzyme necessary for the reaction for forming NAD(P)H from the other components during the course of forming NAD(P)H from the specific component. The mixture is subjected to reaction in an appropriate buffer solution to convert NAD(P)H present in the sample or formed from the other components into NAD(P). G(OX) is used in an amount equivalent to that of NAD(P)H to be eliminated or more, preferably more than 1.5 times the amount of NAD(P)H. Glutathione reductase and the buffer solution are used in concentrations of 0.1 to 20 U/mℓ and 0.001 to 2 M, respectively. The reaction is carried out at a temperature of 25 to 50°C at pH 6 to 9 and is completed in several minutes.

Examples of the buffer are 3-(N-morpholino)propanesulfonic acid (MOPS), N-tris(hydroxymethyl)methyl-2-aminoethanesulfonic acid (TES), Tris-hydrochloride, phosphate, borate, oxalate and Good's buffers.

In the second reaction, G(OX) present in the reaction solution after the first reaction is decomposed by γ-GTP. The decomposition of G(OX) can be accelerated by allowing the mercapto compound to exist. The mercapto compound converts G(OX) into G(OH) and G(OH) is readily decomposed by γ-GTP. The decomposition of G(OX) and G(OH) by γ-GTP is carried out in the presence of glycine, glycylglycine or an equivalent thereof. Examples of the reactions are shown by the reaction formulae (6) and (7) below. The equivalent mentioned above refers to a compound that can react with G(OX) and G(OH) by the action of γ-GTP and thereby can be converted into a substance which does not affect the subsequent quantitative determination of NAD(P)H. Glycylglycine, etc. are used in an amount equivalent to that of G(OX) or more, usually in an amount of 3 to 10 equivalents based on G(OX). The reaction is carried out at a temperature of 25 to 50°C at pH 6 to 9. The mercapto compound is added in an amount equivalent to that of G(OX) or more, preferably in an amount of more than 1.5 equivalents based on G(OX). γ-GTP is used in an amount of 0.1 to 100 U/mℓ. As the mercapto compound, any compound having SH group and which does not inhibit the enzyme used is usable. Examples of the mercapto compound include dithiothreitol (DTT), 2-mercaptoethanol, cysteine or salts thereof, N-acetylcysteine or salts thereof, homocysteine or salts thereof, cysteamine or salts threrof, 2-mercapto-ethanesulfonate, 3-mercapto-1,2-propanediol, 2-mercaptopropionate, 3-mercaptopropionate, mercaptosuccinate, thiomalate, 1-thioglycerine and dithioerythritol.

In cases where the formed NAD(P)H is quantitatively determined by measuring the absorption at 340 nm, it is preferred to used a mercapto compound having no absorption at 340 nm.

As the third reaction, when the specific component is a substrate, the substrate is decomposed to lead to the NAD(P)H-forming reaction and the formed NAD(P)H is quantitatively determined in a conventional manner.

When the specific component is an enzyme, a substrate for the enzyme is added and, if necessary, other enzymes are further added to lead to the NAD(P)H-forming reaction, and the rate of formation of NAD(P)H is determined.

Any known methods and methods which will be developed in the future are applicable to the quantitative determination of NAD(P)H formed by the reaction, unless they are unsuitable for the object of the present invention.

There are known, for example, a method in which the absorption is measured at 340 nm (UV method); the fluorescence method in which the absorption is measured at 365 nm or 460 nm; a method which comprises subjecting NAD(P)H to reaction with diaphorase or phenazine methosulfate, and tetrazolium salt, and measuring the absorption in a visible region; a method which comprises subjecting NAD(P)H to reaction with a chromogen in the presence of peroxidase or thiol oxidoreductase, and diaphorase, and quantitatively determining the dye formed; and a method which comprises converting NAD(P)H into a signal of hydrogen peroxide and quantitatively determining the signal (Japanese Published Unexamined Patent Applications Nos. 43398/85, 180600/85, 205999/84, 106299/84 and 32867/84).

In cases where the sample is a vital component or the like, a variety of surface active agents (for example, nonionic, anionic and cationic surface active agents such as polyoxyethylene higher aliphatic ethers or esters thereof, polyoxyethylene sorbitan higher fatty acid esters, polyoxyethylene-polyoxypropylene copolymer, polyoxyethylene higher aliphatic phenyl ethers, higher fatty acid sodium salts and sodium higher aliphatic benzenesulfonates) and lipases (for example, lipoprotein lipase) are used as lipid-solubilizing agents, etc. In addition, chelating agents (for example, EDTA or salts thereof, ethylene glycol-bis($\beta$-aminoethylether)N,N,N',N'-tetraacetic acid (GEDTA), 1,2-cyclohexane diamine-tetraacetic acid (CYDTA), triethylenetetraamine-hexaacetic acid (TTHA) and iminodiacetic acid (IDA) and enzyme activators (for example, magnesium salts, calcium salts and zinc salts) are appropriately used. Furthermore, if necessary, specific enzyme inhibitors, for example, amylase inhibitor (made from wheat), lipase inhibitor, enzyme inhibitory antibody (monoclonal or polyclonal), and glutathione derivatives which are inhibitors of glutathione reductase may be used in order to terminate the enzyme reaction.

In the case of the aforesaid amylase determination, which is taken as an example of the determination of a component that achieves excellent results by the method of the present invention, addition of NAD(P), ATP, glucokinase (or hexokinase), G6PDH, maltose phosphorylase, glutathione reductase, G(OX) and phosphate to a sample results in formation of NAD(P)H derived from glucose and maltose in the sample and the formed NAD(P)H is converted into NAD(P).

The reactions described above are represented by the reaction formulae (1) through (4) given hereinabove and additionally the following formula:

$$\text{G(OX)} + \text{NAD(P)H} \xrightarrow{\text{Glutathione reductase}} \text{2G(OH)} + \text{NAD(P)} \quad \ldots (5)$$

Then, by addition of maltopentose which is a substrate for amylase, DTT, $\gamma$-GTP and glycylglycine, the remaining G(OX) is converted into G(OH) by the reducing power of DTT and at the same time, G(OX) is converted into glutamylglycylglycine and cystine by $\gamma$-GTP and G(OH) is converted into glutamylglycylglycine and cysteinylglycine.

$$\text{G(OX)} + \text{glycylglycine} \xrightarrow{\gamma\text{-GTP}} \text{Glutamylglycylglycine} + \text{cystine} \quad \ldots (6)$$

$$\text{G(OH)} + \text{glycylglycine} \xrightarrow{\gamma\text{-GTP}} \text{glutamylglycylglycine} + \text{cysteinylglycine} \quad \ldots (7)$$

The added maltopentose is decomposed by amylase and NAD(P)H is formed according to the reaction formulae (1) through (4) shown above. By measuring the absorbancy of the reaction solution at 340 nm, NAD(P)H can be quantitatively determined and accordingly, amylase can be quantitatively determined.

Other examples of the determination of components which achieves excellent results by applying the present invention are shown below.

(1) Quantitative determination of amylase (A) (elimination of maltose and glucose which are originally present)

Maltose which is originally present is decomposed by maltose phosphorylase (MP) in the presence of phosphate. The formed glucose and glucose which is originally present are decomposed by glucose dehydrogenase in the presence of NAD(P), and then the formed NAD(P)H is eliminated. After G(OX) is decomposed, maltopentose is added and decomposed by amylase in the sample. The formed maltose is quantitatively determined.

(2) Quantitative determination of amylase (B) (elimination of maltose which is originally present)

Maltose which is originally present is decomposed in the same manner as in (1). The formed glucose-1-P is converted into glucose- 6-P by phosphoglucomutase. Glucose-6-P is acted on by glucose-6-P-dehydrogenase (G6PDH), and the formed NAD(P)H is eliminated. After G(OX) is decomposed, maltopentose is added and the amylase activity is measured.

(3) Quantitative determination of maltose (elimination of glucose which is originally present)

Glucose which is originally present is decomposed by glucose dehydrogenase in the presence of NAD-(P) , and the formed NAD(P)H is eliminated. After G(OX) is decomposed, MP is added and maltose is quantitatively determined.

(4) Lipase activity (elimination of free glycerol which is originally present)

Free glycerol which is originally present is decomposed by glycerol dehydrogenase (G.DH), and the formed NADH is eliminated. After G(OX) is decomposed, glycerol ester is added and the formed glycerol is quantitatively determined, whereby the lipase activity is determined.

(5) Quantitative determination of glycerol ester (elimination of free glycerol which is originally present)

Free glycerol is decomposed by G.DH, followed by elimination of the formed NADH. After G(OX) is decomposed, lipase is added and the formed glycerol is quantitatively determined, whereby glycerol ester is determined.

(6) Choline esterase activity (elimination of free choline which is originally present)

Free choline is decomposed by choline dehydrogenase and betaine aldehyde dehydrogenase, and the formed NADH is eliminated. After G(OX) is decomposed, choline ester is added and the rate of formation of choline is measured, whereby the choline esterase activity is determined.

(7) Lipase activity (elimination of free fatty acids which are originally present)

Free fatty acids are converted into acyl CoA by CoA and acyl CoA synthetase. The acyl CoA is decomposed by 2-enoylacyl-CoA hydrolyase, L-3-hydroxyacyl-CoA, NAD oxidoreductase, acyl-CoA: acetyl CoA•C-acetyltransferase complex enzyme (HDT) and acyl CoA oxidase, followed by elimination of the formed NADH. After G(OX) is decomposed, glycerol ester is added and the formed fatty acids are quantitatively determined, whereby the lipase activity is determined.

As illustrated above, in cases where the component ($\alpha$) formed during the course of leading the specific component into the NAD(P)H-forming reaction is originally contained in a sample, the specific component in the sample can be determined accurately by leading the component ($\alpha$) originally contained in the sample into the NAD(P)H-forming reaction; eliminating the formed NAD(P)H by the method of the present invention; leading the specific component into the NAD(P)H-forming reaction system; and then determining the formed NAD(P)H.

Certain embodiments of the present invention are illustrated by the following examples.

Example 1 (determination of amylase activity)

Reagent A

| | |
|---|---|
| MOPS buffer solution (Dojin Chemical Research Institute ) pH: 7.5 | 0.1 M |
| Disodium phosphate | 10 mM |
| ATP | 2.5 mg/mℓ |
| Magnesium chloride | 2 mg/mℓ |
| Triton®X-100 | 2 mg/mℓ |
| EDTA | 0.1 mg/mℓ |
| Maltose phosphorylase | 10 U/mℓ |
| Glucokinase | 5 U/mℓ |
| NADP | 1 mg/mℓ |
| G6PDH | 5 U/mℓ |
| GR | 3 U/mℓ |
| G(OX) | 0.5 mg/mℓ |

Reagent B

| | |
|---|---|
| MOPS (pH: 7.5) | 0.1 M |
| Maltopentose | 4 mg/mℓ |
| DTT | 5 mg/mℓ |
| γ-GTP | 10 U/mℓ |
| Glycylglycine | 5 mg/mℓ |

Reagent C

The same composition as that of Reagent A except that GR and G(OX) are not contained.

Reagent D

The same composition as that of Reagent B except that DTT is not contained.

When Reagents A and B are used, NADPH derived from glucose and maltose in a sample is eliminated, followed by measurement of the amylase activity, in which maltopentose acts as a substrate.

When Reagents C and D are used instead of Reagents A and B, NADPH derived from glucose and maltose is not eliminated but remains.

Test 1.

Reagent A (1.5 mℓ) was placed in a cell for a spectrophotometer and heated at 37 °C for 5 minutes. Then, 0.02 mℓ each of samples respectively containing the substances shown in the table was added to the cell, followed by heating for further 5 minutes. Separately, 1.5 mℓ of Reagent B was heated at 37 °C and added to the mixture. Absorption of the reaction solution at 340 nm was traced.

Test 2.

In the procedure of Test 1, Reagents C and D were used instead of Reagents A and B, respectively. The test results show the absorption values of the reaction solutions.

| Sample No. | | Test 1 (ABS/min) | Test 2 |
|---|---|---|---|
| 1<br>2<br>3 | Glucose 10 mg/mℓ<br>Maltose 10 mg/mℓ<br>Amylase 100 mU/mℓ | constant at 0<br>constant at 0<br>increased with time, 0.0041 | constant at 2.2<br>constant at 1.17<br>same as in Test 1 |
| 4 | Glucose 10 mg/mℓ<br>Amylase 100 mU/mℓ | increased with time, 0.0041 | increased with time from 2.3 |
| 5 | Maltose 10 mg/mℓ<br>Amylase 100 mU/mℓ | increased with time, 0.0041 | increased with time from 1.15 |
| 6 | Serum (containing 6.24 mg/mℓ glucose) | increased with time, 0.0025 | increased with time from 1.5 |

The absorption values of Sample Nos. 1 and 2 in Test 2 result from glucose and maltose.

In Sample No. 3, neither glucose nor maltose is originally present and so NADPH to be eliminated is not formed.

In the case of Sample Nos. 4 and 5, after elimination of glucose or maltose which is originally present, maltopentose is decomposed by amylase in the samples and the absorption due to the formation of NADPH increases in Test 1. In Test 2, NADPH derived from glucose and maltose is included from the beginning. The results similar to those with Sample Nos. 4 and 5 are obtained with sample No. 6.

The procedures of Test 1 and Test 2 were carried out using Sample No. 3 with a variety of the amylase activity, i.e. 10 mU/mℓ, 20 mU/mℓ, 50 mU/mℓ, 100 mU/mℓ and 200 mU/mℓ. Reagent B or D was added thereto and the absorbancy was measured after 10 minutes to prepare calibration curves.

The calibration curves are shown in Fig. 1 (Test 1) and Fig. 2 (Test 2).

With respect to the test using Sample No. 6, the absorption was measured 5 minutes after the addition of Reagent B or D (initiation of the absorption due to the amylase reaction) and 10 minutes after the addition and the mean rate of change ($\Delta E$) was calculated to determine the amylase activity. Test 1 and Test 2 were repeated 10 times, respectively, and the coefficients of variation (CV%) obtained are shown in the table below.

Table 1

| Number | Test 1 (U/ℓ) | Test 2 (U/ℓ) |
|---|---|---|
| 1 | 58.6 | 71.8 |
| 2 | 60.7 | 48.7 |
| 3 | 62.5 | 73.7 |
| 4 | 59.8 | 62.6 |
| 5 | 61.6 | 48.2 |
| 6 | 61.8 | 59.4 |
| 7 | 64.0 | 61.6 |
| 8 | 59.5 | 71.2 |
| 9 | 62.5 | 62.5 |
| 10 | 61.4 | 51.7 |
| Mean Value | 61.2 | 61.1 |
| CV (%) | 2.5% | 14.5% |

As is clear from the table, Test 1 provides results much superior to those of Test 2 in reproducibility.

Example 2

The same experiment as in Test 1 of Example 1 was carried out using Sample No. 6, except that 12 U/mℓ glucose dehydrogenase was used instead of ATP, glucokinase and G6PDH. The results are shown below.

Table 2

| Number | Amylase value (mU/mℓ) | | |
|---|---|---|---|
| 1 | | 59.2 | |
| 2 | | 61.9 | |
| 3 | | 62.1 | |
| 4 | | 61.4 | |
| 5 | | 60.2 | |
| 6 | | 61.5 | |
| 7 | | 62.3 | |
| 8 | | 60.0 | |
| 9 | | 61.0 | |
| 10 | | 63.1 | |
| Mean | 61.3 mU/mℓ | | CV = 1.84% |

Example 3

The same experiment as in Test 1 of Example 1 was carried out using Sample No. 6 supplemented with 20 mg/mℓ maltose, except that 5 U/mℓ phosphoglucomutase was used instead of ATP and glucokinase. The results are shown in Table 3.

Table 3

| Number | Amylase value (mU/mℓ) | | |
|---|---|---|---|
| 1 | | 60.4 | |
| 2 | | 59.3 | |
| 3 | | 60.6 | |
| 4 | | 59.5 | |
| 5 | | 63.2 | |
| 6 | | 58.0 | |
| 7 | | 60.1 | |
| 8 | | 63.1 | |
| 9 | | 60.8 | |
| 10 | | 63.0 | |
| Mean | 60.8 mU/mℓ | | CV = 2.77% |

Example 4

The same experiment as in Test 1 of Example 1 was carried out using Sample No. 6, except that 5 U/mℓ hexokinase was used instead of glucokinase. The results are shown in Table 4.

Table 4

| Number | Amylase value (mU/mℓ) | | |
|---|---|---|---|
| 1 | | 60.8 | |
| 2 | | 60.7 | |
| 3 | | 61.7 | |
| 4 | | 61.0 | |
| 5 | | 60.6 | |
| 6 | | 61.2 | |
| 7 | | 61.3 | |
| 8 | | 61.4 | |
| 9 | | 61.4 | |
| 10 | | 61.2 | |
| Mean | 61.2 mU/mℓ | | CV = 0.51% |

Example 5

Reagent A

| | |
|---|---|
| TES buffer solution (pH 6.75) | 0.04 M |
| Pluronic F-68 | 1 mg/mℓ |
| 1,2-Dilinolein | 1.33 mM |
| Co-A | 2 mM |
| Co-lipase | 0.1 mg/mℓ |
| $CaCl_2$ | 1.17 mM |
| NAD | 8.17 mM |
| ATP | 1 mM |
| Acyl CoA synthetase (ACS) | 1.7 U/mℓ |
| Acyl CoA oxidase (ACO) | 20 U/mℓ |
| HDT | 45 U/mℓ |
| $MgCl_2$ | 1.17 mM |
| GR | 5 U/mℓ |
| G(OX) | 0.8 mg/mℓ |

Reagent B

| | |
|---|---|
| TES buffer solution (pH 6.75) | 0.04 M |
| Pluronic® F-68 | 1 mg/mℓ |
| Deoxycholic acid | 16.5 mM |
| Dithioerythritol | 5 mg/mℓ |
| $\gamma$-GTP | 10 U/mℓ |
| Glycylglycine | 8 mg/mℓ |

Serum (0.02 mℓ) having the lipase content of 55.4 mU/mℓ as measured with the lipase assay kit lipase UV (Toyo Jozo Co., Ltd.) was added to 1,5 mℓ of Reagent A. After reaction at 37°C for 3 minutes, Reagent B was added to the reaction mixture. Two minutes after the addition, the rate of increase in absorption of the reaction solution at 340 nm was measured to determine the lipase activity. The mean value of 10 measurements was 56 U/mℓ with CV of 1.8%.

Example 6

The same procedure as in Example 5 was carried out, except that 1-monolinolein was used instead of 1,2-dilinolein and glycerol dehydrogenase was used instead of ACS, ACO, HDT and CoA. Test 1 gave the result of 55.1 U/ℓ with CV of 2.7%.

## Claims

1. A method for quantitative determination of NAD(P)H derived from a specific component, which comprises converting NAD(P)H present in a sample or formed from components other than the specific component by reactions into NAD(P) by the action of glutathione of oxidation type and glutathione reductase (first reaction); decomposing the remaining glutathione of oxidation type by the action of γ-glutamyl transpeptidase in the presence of glycine, glycylglycine or an equivalent thereof (second reaction); forming NAD(P)H from the specific component in the sample utilizing an NAD(P)H-forming reaction system; and quantitatively determining the formed NAD(P)H.

2. A method according to claim 1, wherein said quantitative determination of NAD(P)H is effected by measuring the ultraviolet absorption of a solution containing NAD(P)H at 340 nm.

3. A method according to claim 1, wherein said reactions are carried out in a buffer solution.

4. A method according to claim 1, wherein NAD(p)H derived from the specific component stoichiometrically reacts to form hydrogen peroxide and the hydrogen peroxide is quantitatively determined.

5. A method according to claim 1, wherein said specific component is selected from amylase, maltose, lipase, glycerol ester and choline esterase.

6. A method according to Claim 1, wherein said second reaction is carried out in the presence of a mercapto compound.

7. A method according to claim 6, wherein said mercapto compound is selected from dithiothreitol (DTT), 2-mercaptoethanol, cysteine or salts thereof, N-acetylcysteine or salts thereof, homocysteine or salts thereof, cysteamine or salts thereof, 2-mercapto-ethanesulfonate, 3-mercapto-1,2-propanediol, 2-mercaptopropionate, 3-mercaptopropionate, mercaptosuccinate, thiomalate, 1-thioglycerine and dithioerythritol.

8. A method according to claim 1 wherein said specific component is amylase and which comprises the steps of:
   (1) subjecting the sample to reaction with NAD(P), ATP, glucokinase, glucose-6-P-dehydrogenase, maltose phosphorylase, glutathione reductase, glutathione of oxidation type and phosphate;
   (2) subjecting the reaction solution to reaction with maltopentose, dithiothreitol, γ-glutamyltranspeptidase and glycylglycine; and
   (3) measuring the absorption of the reaction solution at 340 nm.

## Patentansprüche

1. Verfahren zur quantitativen Bestimmung von NAD(P)H, das von einer spezifischen Komponente stammt, umfassend die Umwandlung von NAD(P)H, das in einer Probe vorliegt oder das von anderen Komponenten als der spezifischen Komponente erzeugt wird, durch Umsetzungen zu NAD(P) unter Wirkung von Glutathion vom Oxidationstyp und Glutathionreduktase (erste Reaktion), Zersetzung des verbleibenden Glutathions vom Oxidationstyp durch die Wirkung von γ-Glutamyltranspeptidase in Gegenwart von Glycin, Glycylglycin oder einem Äquivalent davon (zweite Reaktion), Erzeugung von NAD(P)H aus der spezifischen Komponente in der Probe unter Verwendung eines NAD(P)H erzeugenden Reaktionssystems und quantitative Bestimmung des erzeugten NAD(P)H.

2. Verfahren nach Anspruch 1, wobei die quantitative Bestimmung von NAD(P)H durch Messung der UV-Absorption einer NAD(P)H enthaltenden Lösung bei 340 nm erfolgt.

3. Verfahren nach Anspruch 1, wobei die Reaktionen in einer Pufferlösung durchgeführt werden.

4. Verfahren nach Anspruch 1, wobei NAD(P)H, das von der spezifischen Komponente stammt, stöchiometrisch unter Erzeugung von Wasserstoffperoxid reagiert und das Wasserstoffperoxid quantitativ bestimmt wird.

5. Verfahren nach Anspruch 1, wobei die spezifische Komponente ausgewählt ist aus Amylase, Maltose, Lipase, Glycerinester und Cholinesterase.

6. Verfahren nach Anspruch 1, wobei die zweite Reaktion in Gegenwart einer Mercaptoverbindung durchgeführt wird.

7. Verfahren nach Anspruch 6, wobei die Mercaptoverbindung ausgewählt ist aus Dithiothreit (DTT), 2-Mercaptoethanol, Cystein oder Salzen davon, N-Acetylcystein oder Salzen davon, Homocystein oder Salzen davon, Cysteamin oder Salzen davon, 2-Mercaptoethansulfonat, 3-Mercapto-1,2-propandiol, 2-Mercaptopropionat, 3-Mercaptopropionat, Mercaptosuccinat, Thiomalat, 1-Thioglycerin und Dithioerythrit.

8. Verfahren nach Anspruch 1, wobei die spezifische Komponente Amylase ist und das Verfahren die folgenden Schritte umfaßt:
(1) Umsetzung der Probe mit NAD(P), ATP, Glucokinase, Glucose-6-P-Dehydrogenase, Maltosephosphorylase, Glutathionreduktase, Glutathion vom Oxidationstyp und Phosphat;
(2) Umsetzung der Reaktionslösung mit Maltopentose, Dithiothreit, γ-Glutamyltranspeptidase und Glycylglycin; und
(3) Messung der Absorption der Reaktionslösung bei 340 nm.

## Revendications

1. Procédé de dosage de NAD(P)H dérivant d'un composant particulier intéressant, qui comprend le fait de convertir le NAD(P)H présent dans un échantillon ou formé à partir de composants autres que le composant intéressant, selon certaines réactions, en NAD(P), grâce à l'action de glutathion oxydé et de glutathion réductase (première réaction), le fait de décomposer le glutathion oxydé résiduel grâce à l'action de γ-glutamyl transpeptidase, en présence de glycine, de glycylglycine ou d'un équivalent de ceux-ci (seconde réaction), le fait de former du NAD(P)H à partir du composant intéressant présent dans l'échantillon en mettant à profit un système de réactions formant du NAD(P)H, et le fait de doser le NAD(P)H formé.

2. Procédé conforme à la revendication 1, dans lequel on effectue le dosage de NAD(P)H en mesurant l'absorption de rayonnement ultraviolet à 340 nm par la solution contenant le NAD(P)H.

3. Procédé conforme à la revendication 1, dans lequel lesdites réactions sont effectuées dans une solution tampon.

4. Procédé conforme à la revendication 1, dans lequel on fait stoechiométriquement réagir le NAD(P)H dérivant du composant intéressant, pour former du peroxyde d'hydrogène que l'on dose.

5. Procédé conforme à la revendication 1, dans lequel ledit composant intéressant est choisi parmi l'amylase, le maltose, la lipase, un ester du glycérol et la choline estérase.

6. Procédé conforme à la revendication 1, dans lequel ladite seconde réaction est effectuée en présence d'un thiol.

7. Procédé conforme à la revendication 6, dans lequel ledit thiol est choisi parmi le dithiothréitol (DTT), le 2-mercaptoéthanol, la cystéine et ses sels, la N-acétylcystéine et ses sels, l'homocystéine et ses sels, la cystéamine et ses sels, le 2-mercaptoéthane-sulfonate, le 3-mercapto-1,2-propanediol, le 2-mercaptopropionate, le 3-mercaptopropionate, le mercaptosuccinate, le thiomalate, la 1-thioglycérine et le dithioérythritol.

**8.** Procédé conforme à la revendication 1, dans lequel ledit composant intéressant est l'amylase, et qui comprend les étapes consistant à :

(1) faire réagir l'échantillon avec du NAD(P), de l'ATP, de la glucokinase, de la glucose-6-P déshydrogénase, de la maltose phosphorylase, de la glutathion réductase, du glutathion oxydé et du phosphate ;

(2) faire réagir la solution réactionnelle avec du maltopentose, du dithiothréitol, de la $\gamma$-glutamyl-transpeptidase et de la glycylglycine ; et

(3) mesurer l'absorption de la solution réactionnelle à 340 nm.

FIG.1

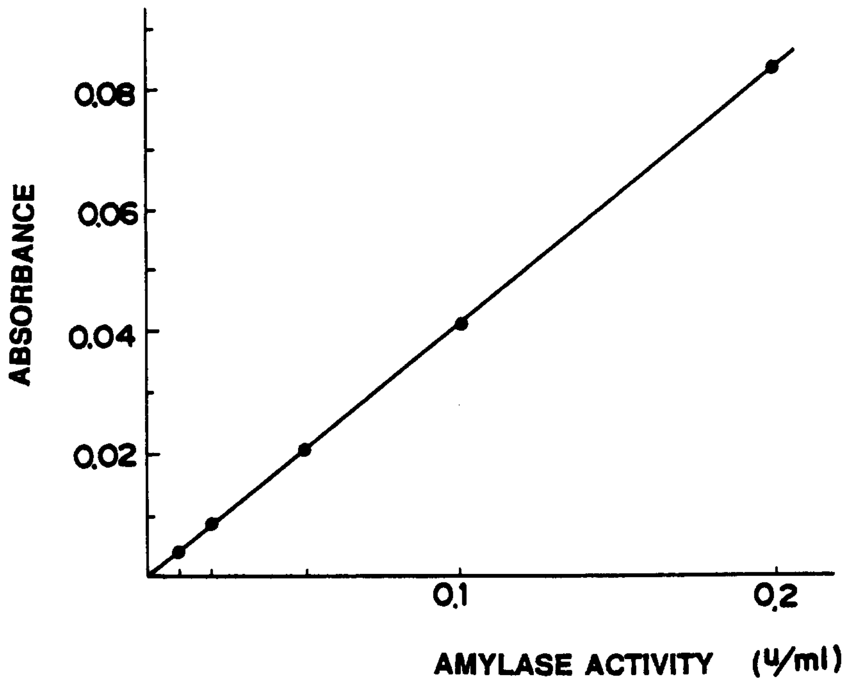
ABSORBANCE
0.08
0.06
0.04
0.02
0.1
0.2
AMYLASE ACTIVITY (U/ml)

FIG.2

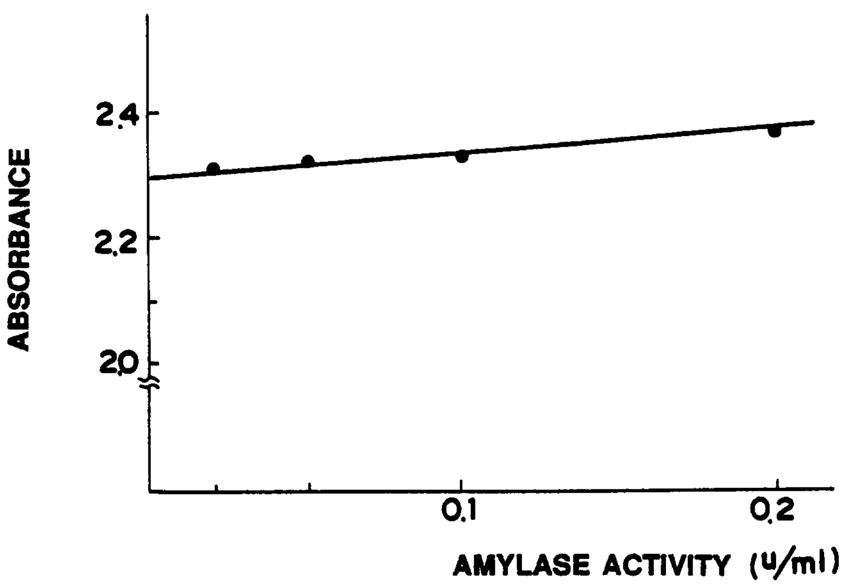
ABSORBANCE
2.4
2.2
2.0
0.1
0.2
AMYLASE ACTIVITY (U/ml)